# EUROPEAN PATENT APPLICATION

(11) **EP 1 266 624 A1**
(43) Date of publication of application: **18.12.2002**
(21) Application number: 02012990.4
(22) Date of filing: 12.06.2002
(51) Int. Cl.: A61B 10/00

(54) **Biopsy needle**

(30) Priority: 12.06.2001 JP 2001176549
(71) Applicant: H.S. Hospital Service S.p.a., 00040 Pomezia (Roma) (IT)
(72) Inventor: Yukio Kawashima, Tochigi-Ken (JP)
(74) Representative: Crugnola, Pietro

(57) **Abstract**

A biopsy needle (10) comprised of an inner needle (11; 26) formed with a notched part (lla) in its front end, and a hollow outer needle (12) in which the inner needle (11; 26) in inserted. An inner hole (11b) is formed in an axial direction from a rear end of the inner needle (11; 26) to the notched part (11a). The front end of the inner needle (11; 26) is inserted in tissue, and the outer needle (12) is then moved to cover the inner needle (11; 26). Due to this, the tissue is sampled and cut to fill the notched part (11a) of the inner needle (11; 26). A blood coagulant or other liquid medicine can be inserted from a receiver (21), connected to the inner hole (11b), and supplied into the human body through the inner needle (11; 26). A vacuum creating device may be connected with the inner hole (11b) in order to make sure that a portion of tissue enters the notched part (lla) of the inner needle (11; 26) and adheres to that notched part (lla) before being cut away by the outer needle (12).

## Description

### BACKGROUND OF THE INVENTION

### 1. Field of the Invention.

The present invention relates to a bioprobe far sampling tissue of the human body.

### 2. Description of the Related Art.

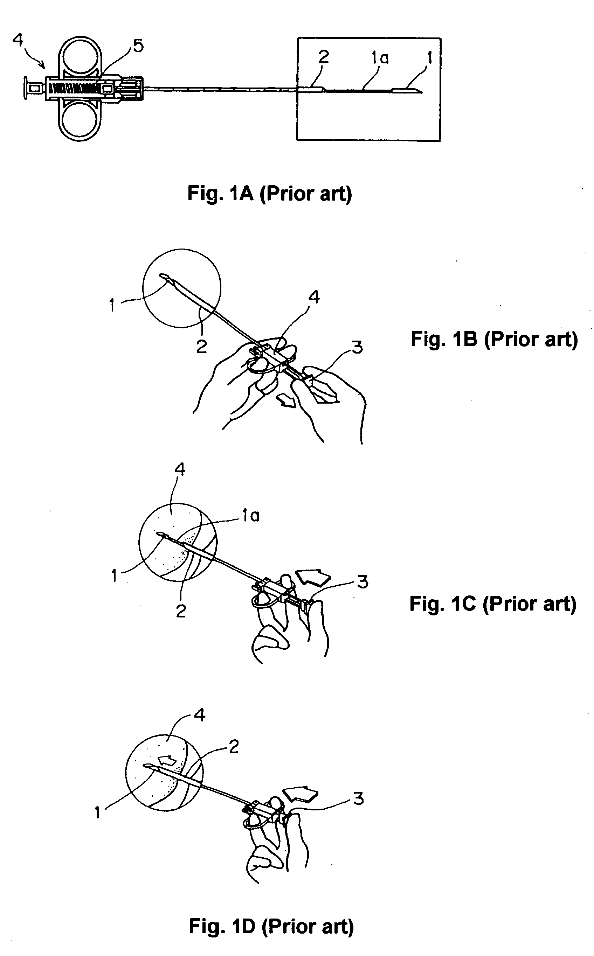

As a conventional biopsy needle, the example shown in Figs. 1A to 1D is well known. As shown in Fig. 1A, the biopsy needle has an inner needle 1 formed with a notched part 1a at its front end, and a hollow outer needle 2, in which the inner needle 1 is inserted. The procedure for use of this biopsy needle as follows:

First, a plunger 3 is pulled back to set the inner needle 1 and outer needle 2 (Fig. 1B). As a result, a coil spring 5 becomes compressed. In this compressed state, the inner needle and outer needle are inserted into the tissue T of the body. The plunger 3 is then pushed in, whereupon only the inner needle 1 in inserted into the tissue T of the body (Fig.1C), and part of the tissue T enters in the notched part 1a of the inner needle 1. When the plunger 3 is further pushed in, the setting of the coil spring 5 is released. As shown in Fig. LD, the outer needle 2 is pushed out by the return force of the coil spring 5, whereby the part of the tissue T, which had entered the notched part 1a, is cut out by the outer needle 2.

In summary, the problem to be solved by this invention is that the conventional biopsy needles do not guarantee that a part of the issue T enters the notched part of the inner needle, with the consequence that biopsy must often be repeated; in addition conventional biopsy needles do not have the function of injecting liquid medicines at the concerned location or removing body fluids and tissues using the inner needle or outer needle inserted into the body.

### SUMMARY OF THE INVENTION

An object of the present invention is to provide a biopsy needle improved in functionality which can guarantee that part of the tissue T to be sampled enters the notched part of the inner needle and can use the inner needle or outer needle to inject liquid medicines or sample body fluid or tissue.

According to the present invention, there is provided a biopsy needle comprised of an inner needle formed with a notched part in its front end and a hollow outer needle in which the inner needle is inserted. The biopsy needle samples tissues by inserting the front end of the inner needle in the tissue, then making the outer needle move to cover the inner needle and cut the tissue. This procedure partially fills the notched part of the inner needle with tissue. In the inner needle, an inner hole is formed in an axial direction from the rear end of the inner needle to the notched part.

### BRIEF DESCRIPTION OF THE DRAWINGS

The objects and advantages of the present invention will be better understood from the following description, with reference to the accompanying drawings in which:
Figs 1A to 1D are views of a biopsy needle of a prior art;
Fig. 2 is a perspective view of a first embodiment of a biopsy needle according to the present invention;
Figs. 3A to 3C are a plan view, side view, and sectional view of a base plate of the biopsy needle;
Figs 4A and 4B are a plan view and side view of a movable base;
Fig. 5 is a side view of an inner needle and handle;
Fig. 6 is a sectional view of the inner needle and handle;
Fig. 7 is an enlarged detail of Fig. 6;
Fig. 8 is another enlarged detail of Fig. 6.
Fig. 9 is a view from above of Fig. 8.

### DESCRIPTION OF THE PREFERRED EMBODIMENTS

The present invention will be described below with reference to the embodiments shown in the drawings.

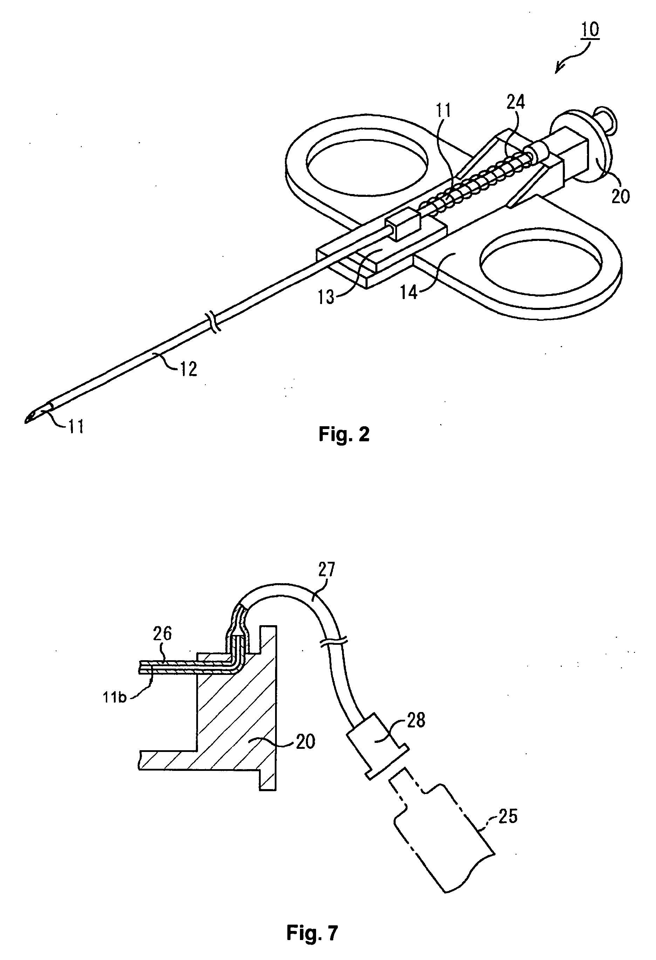

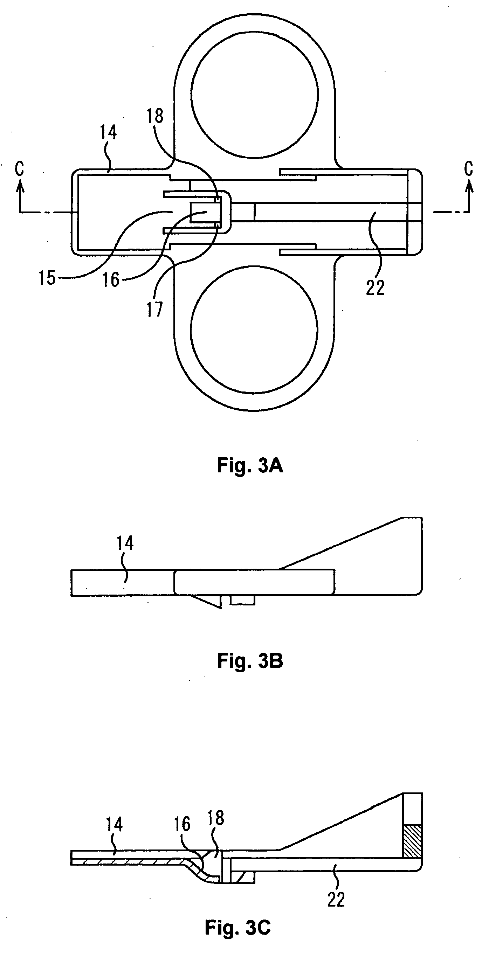

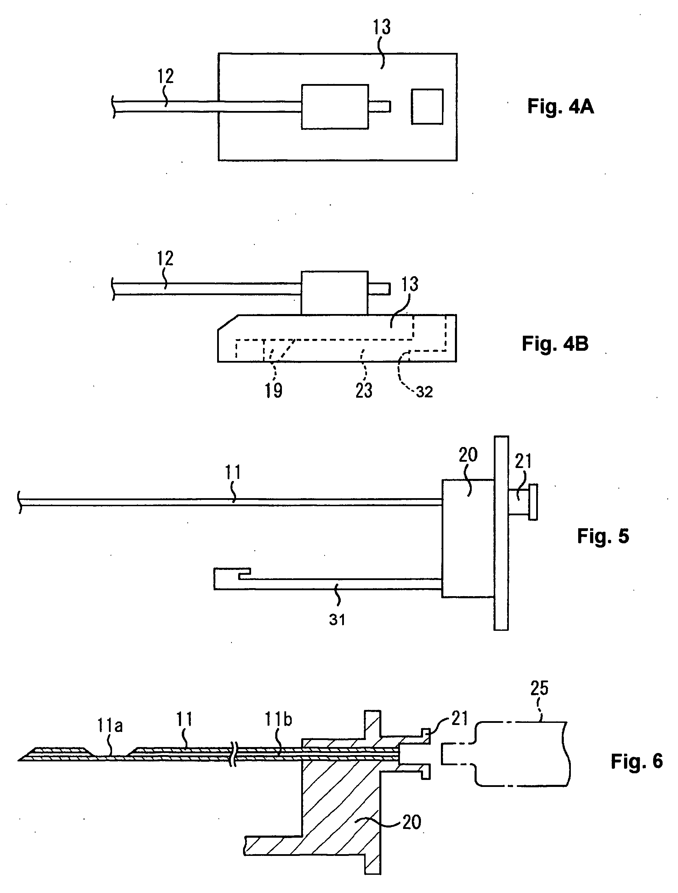

Fig. 2 is a perspective view of a first embodiment of the present invention. A biopsy needle 10 is provided with an inner needle 11 and a hollow outer needle 12, in which the inner needle 11 is inserted. The outer needle 12 is fixed to a movable base 13, which is movably supported by a base plate 14. Fig. 2 shows the state of the biopsy needle 10 with the cover covering the base plate 14 removed. The base plate 14, as shown in Figs 3A, 3B and 3C, has a spring plate 15, and claws 17 and 18 are formed on the two sides of the front end of the spring plate 15. The claws 17 and 18 straddle an inclined surface 16. On the other hand, the movable base 13, as shown in Fig. 4B, has a claw 19 engaging with the claws 17 and 18.

The inner needle 11 is fixed to a handle 20, and has an inner hole 11b extending in an axial direction from a front end to a rear end of the inner needle 11. The rear end of the inner needle 11 is formed with a receiver 21 for connecting the inner hole 11b with the outside. The inner hole 11b is formed at least from the rear end of the inner needle 11 to the notched part 11a, but as shown in Fig. 6, it may also be formed to the front end of the inner needle 11.

When the inner hole 11b is formed to the front end of the inner needle 11, the notched part 11a of the inner needle 11 shows a groove 40 at its bottom portion, said groove communicating with the portions of the inner hole 11b in the rear end and front end of the inner needle, respectively.

The handle 20 has a hook 31 attached to it (Fig. 5). The hook 31 passes through a groove 22 of the base plate 14, and engages with a bottom groove 23 of the movable base 13.

The operation of the biopsy needle will be explained below.
Fig. 2 shows the initial state of the biopsy needle 10. At this time, the inner needle 11 in inserted in the outer needle 12, and the movable base 13 is biased forward (left direction in Fig. 2) by a coil spring 24. When the handle 20 is pulled, the hook 31 engages with a step portion 32 at the movable base 13 to move the movable base 13 backward (right direction in Fig. 2) against the force of the coil spring 24. As a result, the claw 19 of the movable base 13 moves from the front side F (Fig. 3C) of the claws 17 and 18 to the rear side R (Fig. 3C) thereof. Thus, the claw 19 engages with rear faces 17a and 18a (Figs. 3A and 3C) of the claws 17 and.18 by the coil spring 24, and is locked at that position, whereby the coil spring 24 is kept in the compressed state.

In this state, the inner needle 11 and the outer needle 12 are inserted together into the tissue of the body. Next, the handle 20 is pushed, whereupon just the inner needle 11 is inserted into the tissue of the body.

At this time, if necessary to sample body fluid around the tissue, the front end of a syringe 25 is inserted into the receiver 21 of the handle 20 to suck up and obtain the body fluid.

When the handle 20 is pushed further, the front end of the hook 31 presses against the inclined surface 16 of the spring plate 15. Thus, the claws 17 and 18 are lowered, so that the engagement with the claw 19 is released. The outer needle 12 is then pushed out by the return force of the coil spring 21, and the part of the tissue, which had entered the notched part 11a, is cut away by the outer needle 12.

A blood coagulant or other liquid medicine is then inserted from the receiver 21. When cutting tissue with the outer needle 12, however, the piece of tissue does not. fill the notched part 11a completely, but is pressed to the front end of the outer needle and is mostly at the front. That is, space is formed at the rear of the notched part 11a. Therefore, it is possible to inject a blood coagulant or other liquid medicine into that space. In a conventional biopsy needle, the inner needle is completely pulled out from the outer needle in order to inject a liquid medicine through the outer needle. When doing this, however., there is the problem of blood flowing out backward from the outer needle. When using the biopsy needle of the present invention, however, there is the remarkable effect where it is possible to inject liquid medicine into the body in the state where the inner needle is still inserted and therefore it is possible to prevent the backward flow of blood from the outer needle.

Finally, the inner needle and outer needle are both pulled out and the piece of tissue taken out.

Before pushing out the outer needle 12, it is advisable to create a vacuum into the hole 11b of the inner needle 11, by connecting the receiver 21 of the handle 20 to a vacuum creating device (not shown), for instance a syringe. Creating a vacuum into the hole 11b makes sure that a portion of tissue enters the notched part 11a of the inner needle 11 and adheres to that notched part before being cut away by the outer needle 12. The adhesion of the portion of tissue to the notched part 11a is improved if the hole 11b is formed to the front end of the inner needle 11, forming the groove 40 at the bottom portion of the notched part 11a of the inner needle 11. In fact, the vacuum created into the hole 11b exerts a suction force on the rear and bottom sides of the portion of tissue entered into the notched part 11a causing that portion of tissue to adhere firmly to rear and bottom sides of the notched part 11a, so that the portion of tissue can not escape from the notched part 11a before the outer needle 12 is advanced to cut away the tissue. In order to facilitate creation of vacuum, it is preferred that the ratio between the outer diameter of the inner needle 11 and the diameter of the inner hole 11b is comprised between 6 and 8.

The same vacuum creating device, for instance a syringe, used for creating vacuum into the hole 11b may be then used to inject blood coagulant or other liquid medicine, as described above.

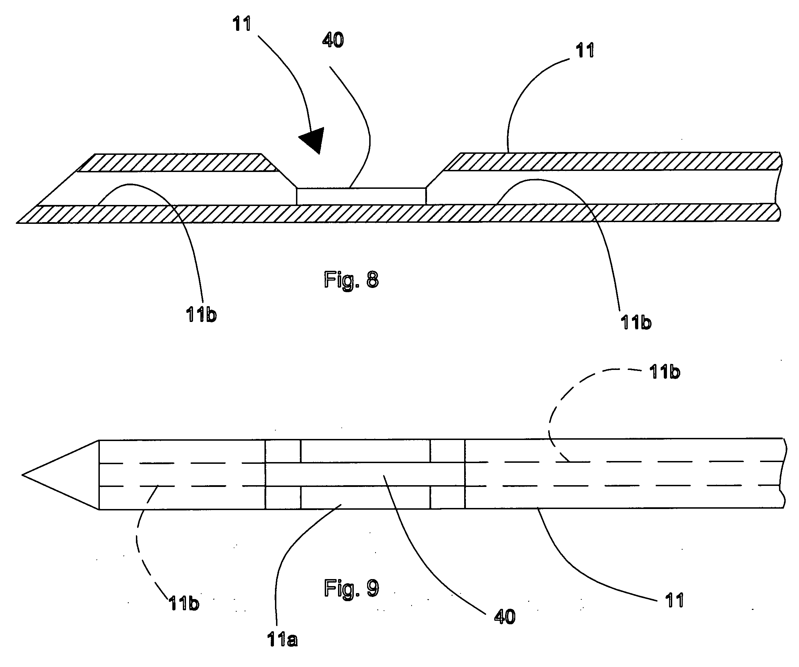

Fig. 7 shows a handle of a second embodiment of the biopsy needle.
In this embodiment, the inner needle 26 is bent midway without being passed through the handle 20 and is connected with a tube 27. A connector 28 is attached to the rear end of that tube 27. A syringe 25 can be connected to the connector 28. Due to this, as shown in Fig. 7, the rear surface of the handle 20 becomes completely flat and the inner needle 26 can be easily pushed when being inserted, so the operability is improved. Further, since the tube 27 is used for connecting to the syringe 25, the movement of the syringe 25 is not transmitted to the inner needle 26, so the biopsy needle will not be moved by the syringe 26. For example, it is then possible to operate the handle 20 with the left hand and connect the syringe 25 to connector 28 and inject liquid medicine with the right hand. This improves the operability.

As described above, according to the first and second embodiments, it is possible to improve the function of the biopsy needle by forming an inner hole in the axial direction of the inner needle.

Although the embodiments of the present invention have been described herein with reference to the accompanying drawings, obviously many modifications and changes may be made by those skilled in this art without departing from the scope of the invention.
The present .disclosure relates to subject matter contained in Japanese Patent Application No.2001-176549 (filed on June 12, 2001), which is expressly incorporated herein, by reference, in its entirety.

## Claims

1. A biopsy needle (10) comprising:
an inner needle (11; 26) that has a notched part (11a) in its front end, and an inner hole (11b) extending in an axial direction at least from a rear end of said inner needle (11) to said notched part (11a); and
a hollow outer needle (12) in which said inner needle (11) is inserted, said hollow outer needle (12) being movable relative to said inner needle (11; 26).

2. A biopsy needle according to claim 1, wherein the front end of said inner needle (11; 26) is inserted in tissue, and said outer needle (12) is moved to cover said inner needle (11; 26) so that said tissue is sampled and cut to. partially fill said notched part (11a).

3. A biopsy needle according to claim 1, or 2, wherein said inner hole (11b) extends in said axial direction from said rear end to the front end of said inner needle (11; 26).

4. A biopsy needle according to claim 3, wherein a groove (40) is provided in the bottom portion of said notched part (11a), said groove (40) communicating with said inner hole (11b).

5. A biopsy needle according to one of preceding claims, wherein said rear end of said inner needle (11) has a receiver (21) for connecting said inner hole (11b) with the outside thereof.

6. A biopsy needle according to claim 1, wherein said inner needle (26) is bent midway and is connected with a tube (27), to which a syringe (25) or a vacuum creating device can be connected.

7. A biopsy needle according to one of preceding claims, wherein the ratio between the outer diameter of said inner needle (11; 26) and the diameter of said inner hole (11b) is comprised between 6 and 8.
